# EUROPEAN PATENT SPECIFICATION

(11) **EP 0 394 394 B1**
(45) Date of publication and mention of the grant of the patent: **18.01.1995**
(21) Application number: 89910615.7
(22) Date of filing: 26.09.1989
(51) Int. Cl.: C07F 9/58, C07F 9/38, C07C 229/16, A61K 49/00, C07D 257/02, C07D 273/00

(54) **MANGANESE(II) CHELATES**
MANGAN-CHELATE
CHELATES DE MANGANESE (II)

(30) Priority: 27.09.1988 US 249744
(43) Date of publication of application: 31.10.1990
(73) Proprietor: NYCOMED SALUTAR, INC., Sunnyvale, California 94086 (US)
(72) Inventor: ROCKLAGE, Scott, Los Gatos, CA 95032 (US); CACHERIS, William, Sunnyvale, CA 94086 (US); JAMIESON, Gene, Boulder Creek, CA 95006 (US)
(74) Representative: Cockbain, Julian, Dr.
(86) International application number: PCT/EP89/01122
(87) International publication number: WO 90/03975

(56) References cited:
- EP-A- 0 232 751
- EP-A- 0 250 358
- EP-A- 0 255 471
- EP-A-02 927 61
- DE-A- 3 401 052
- DE-A- 3 427 980
- US-A- 4 181 672
- US-A- 4 322 361

## Description

This invention relates to novel protonated manganese (II) chelates, to low osmolarity magnetic resonance imaging (MRI) contrast media containing or prepared with them and to methods for their preparation.

In diagnostic imaging, contrast agents are frequently used to enhance the image contrast, for example between different organs or between healthy and unhealthy tissue within the same organ. The nature and manner of operation of the contrast agents depend upon the nature of the imaging technique and the organ or tissue which is to be imaged.

Thus in magnetic resonance imaging (MRI), image contrast may be enhanced by introducing into the body zone being imaged a contrast agent which affects the nuclear spin reequilibration characteristics of the nuclei (generally water protons in body tissues or fluids) which are responsible for the magnetic resonance (MR) signals from which the MR images are generated.

In 1978 Lauterbur (see Lauterbur et al., pages 752-759 in "Electrons to Tissues - Frontiers of Biological Energetics", Vol. 1, edited by Dutton et al., Academic Press, NY, 1978) proposed the use of paramagnetic metal ions, such as Mn(II), as MRI contrast agents.

Mn(II) chelates are particularly useful as MRI contrast agents because manganese, being normally present in the body in low concentrations, is less toxic than many paramagnetic metal ions, and because Mn(II) is particularly effective at enhancing MRI contrast. A number of Mn(II) chelates have been investigated for use as MRI contrast agents and are reported in the literature.

Chelates of paramagnetic metal ions are normally formed by reacting an aqueous solution of a paramagnetic metal salt with the chelating agent in approximately equimolar proportions to yield an acidic solution of the chelate and salt anion. An alkaline agent such as sodium hydroxide is added to neutralize the solution to a physiologically acceptable pH, introducing further ions (cations).

Thus for example US-A-4647447 (Schering AG) describes the preparation of Mn(II) chelates of diethylenetriaminepentaacetic acid (DTPA), trans-1,2-diamino-cyclohexane-N,N,N',N'-tetraacetic acid (DCTA), ethylenediaminetetraacetic acid (EDTA), and a variety of other chelating agents. Also disclosed is the preparation of paramagnetic metal ion chelates of ethylenedinitrilotetrakis(methylphosphonic acid) (EDTP) and 1,4,7,10-tetraazacyclododecanetetraacetic acid (DOTA). The methods described in this patent for preparing Mn(II) chelates comprise the reaction of MnCO₃, a water-soluble manganese salt, with the chelating agent in aqueous solution to yield a chelate solution containing the carbonate ion. Sodium hydroxide is added to raise the pH to 7. The solution is then generally evaporated to dryness. In Example 13 of US-A-4647447 MnCO₃ is reacted with ethylenedinitrilotetra(acetohydroxamic acid), acetone is added, and after several hours, a precipitated crystallizate of Mn(II) complex is removed, washed and dried at 50°C in vacuo to yield the dihydrate. In Example 52, MnCO₃ is reacted with DTPA, and the solution is heated to 95°C, neutralized with sodium hydroxide solution, filtered and put into ampoules. The preparation of a lipid conjugate and its purification using a Sephadex G50 gel filtration column (Sephadex is a Trade Mark) is also described in Example 57 of this patent.

Such processes yield a hyperosmotic injection solution resulting in an increased LD₅₀ for the drug product. Substantial efforts would then be required to separate the chelate from the soluble ions, in order to lower the toxicity of the final product formulation.

It may also be noted that where Mn(II) chelates have previously been prepared as part of studies of the chelating tendencies of metal ions, reagent grade nitrates and chlorides have been used. See for example Frost, A. et al, J.Am.Chem.Soc. 80:530 (1958); L'Eplattenier, F. et al, J.Am.Chem.Soc. 89:837 (1967)).

A need exists for improved MRI contrast media and it is an objective of the present invention to develop novel Mn(II) chelates suitable for the preparation of low osmolarity contrast media.

Thus the present invention provides a process for the preparation of a Mn(II) chelate, which process comprises reacting MnO in an aqueous medium, e.g. water, at a pH of no more than 5 and a temperature of from 20 to 50°C with a chelating compound other than EDTA which chelating compound is substantially water-insoluble and is substantially protonated whereby at least 75% of the labile protons in said chelating compound in its fully protonated form are present, and optionally subsequently dissolving precipitated Mn(II) chelate in an aqueous base, preferably a physiologically tolerable aqueous base solution.

The present invention is based on the surprising discovery that a Mn(II) chelate may be formed by reacting an essentially water insoluble manganese compound, manganese(II) mono-oxide (MnO), with an essentially water-insoluble form of the chelating agent; it had previously been thought that a soluble form of Mn(II) (i.e. a salt) was required for chelation, thereby necessitating the introduction of undesired counteranions.

The term "substantially protonated" as used herein indicates that protons represent at least 75% of the labile cations in the chelate product or chelating compound. In preferred embodiments, the chelate of chelating compound is fully protonated i.e. protons represent 100% of the cations therein. Most preferably, the protons are acidic protons. It will therefore be realized that the Mn(II) chelate of the invention may be a neutral species or may be in the form of a salt of a chelate ion with a, preferably physiologically tolerable, counterion.

The chelates of the invention may be formed by reacting MnO with an aqueous suspension comprising a molar equivalent or molar excess of the chelating compound. When the reaction is carried out at a pH of 3 to 5 a fully protonated Mn(II) chelate precipitates. This precipitated, fully protonated chelate may if desired be converted into a soluble salt form, e.g. by reaction with a base for example to remove one or more labile acid protons.

MRI contrast media can be formulated from the solid chelates by dissolving the precipitated chelate in an aqueous solution containing 1 to 6, preferably 1 to 3, molar equivalents of pharmaceutically acceptable, non-toxic inorganic and/or organic base. Suitable bases include those having as cations lithium, sodium and especially calcium ions. Suitable organic bases include ethanolamine, diethanolamine, morpholine, glucamine, N,N-dimethylglucamine and N-methylglucamine, for example. The solutions can be formulated at any temperature, but at temperatures of 20 to 40°C, the dissolution rates are most efficient.

The essential step in the process of the invention is the reaction of MnO, initially in a particulate form, with an aqueous suspension of the chelating compound. The chelating compound is conveniently present, preferably in a fully protonated or free acid form, in equimolar proportions (1:1) or a molar excess to ensure solubilization of all of the MnO. Preferably, the MnO and chelating compound are reacted in equimolar proportions. The reaction is carried out at a temperature of from 20 to 50°C, preferably from 20 to 30°C. The reaction proceeds more rapidly at the elevated temperatures.

The reaction in aqueous suspension at high concentration and near boiling temperature of MnO with soluble chelating agents which may at least initially be present above their solubility limits is known - there however any undissolved chelating agent serves as a reservoir that is drawn on throughout the reaction. (See for example EP-A-255471, EP-250358, US-A-4322361 and DE-A-3401052).

In the process of the invention the reaction is carried out at a pH of not more than 5, e.g. 3-5, or even less than 3, and a precipitate is formed which can easily be removed from the solution. A reaction time of from 8 to 10 hours is usually sufficient for completion of the reaction at temperatures above 20°C, and the fully protonated Mn(II) chelate product precipitates immediately from the acidic solution. It can be separated from the reaction mixture, washed, dried, and stored for later preparation of the MRI contrast media.

Any chelating compound which forms a stable chelate with the Mn(II) ion may be used in the process of the invention, although those having acidic protons are preferred. Suitable compounds include those of Formula I, 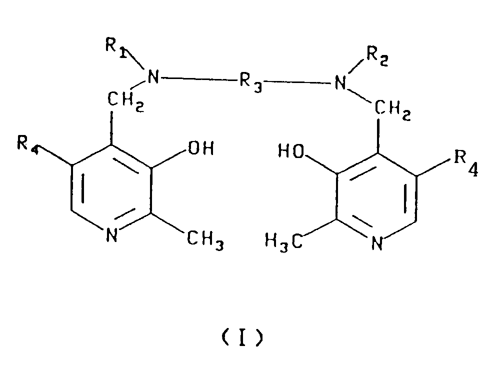
(wherein
R₁ represents a hydrogen atom or a group 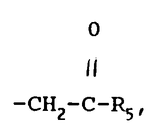
and
R₂ represents a hydrogen atom or a group 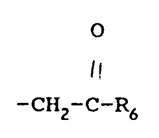
with the proviso that one of the groups R₁ and R₂ is other than hydrogen;
R₃ represents a C₁₋₈ alkylene, C₅₋₈ 1,2-cycloalkylene, or C₆₋₁₀ 1,2-arylene group;
each of the groups R₄ which may be the same or different represents a hydrogen atom, a C₁₋₆ alkyl group or a group -CH₂OH, or a group 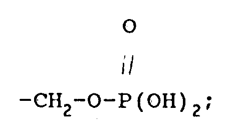
with the proviso that where one R₄ represents hydrogen or alkyl the other represents a group -CH₂OP(O)(OH)₂;
R₅ and R₆ which may be the same or different each represents a hydroxy, C₁₋₁₈, optionally hydroxy-substituted, alkoxy, amino or C₁₋₈ alkylamino group),
and pharmaceutically acceptable salts, and where group R₄ represents a group CH₂-O-PO(OH)₂, the phosphate group mono and diesters thereof with mono and polyhydric C₁₋₁₈ alkyl- or alkylamino alcohols.

Preferred chelating compounds include compounds of Formula I wherein R₅ and R₆ each individually represent C₁₋₈ alkoxy, hydroxyethoxy, dihydroxypropoxy (i.e. residues of ethylene glycol or glycerol respectively), amino or C₁₋₈ alkylamino groups, or especially preferably R₅ and R₆ each represent a hydroxy group and the salts of such compounds.

Compounds of Formula I and processes for their preparation are described in EP-A-290047 (Salutar Inc) and EP-A-292761 (Salutar Inc.).

The term "alkyl" and "alkylene", as used herein, include both straight and branch-chained, unsaturated and, particularly, saturated hydrocarbon structures. The term "1,2-cycloalkylene" includes both cis and trans cycloalkyl groups and alkyl substituted cycloalkylene groups bonded at the 1,2-positions to respective nitrogen atoms and alkyl substituted derivatives thereof having from 3 to 8 carbons. The term "1,2-arylene" includes phenyl, pyridyl and naphthyl groups bonded at the 1,2-positions to respective nitrogen atoms and C₃₋₁₀ alkyl substituted derivatives thereof.

Since not all of the acidic protons of the chelates are substituted by the central paramagnetic ion, the solubility of the chelate can be increased if a number of the remaining protons are converted to salts of the conjugate base with physiologically biocompatible cations of inorganic and/or organic bases or basic amino acids. For example, lithium, sodium and especially calcium ions are suitable inorganic countercations. Suitable organic cations include, for example, those of organic bases such as ethanolamine, diethanolamine, morpholine, glucamine, N,N-dimethylglucamine, N-methylglucamine. Ions of amino acids such as lysine, arginine and orithine are also suitable counter cations as generally are those of other bases or naturally occuring acids.

By way of example suitable chelating compounds of Formula I include
N,N'-bis(pyridoxal-5-phosphate)-1,3-(n-propylene)-N,N'-diacetic acid;
N,N'-bis(pyridoxal-5-phosphate)-1,2-(n-propylene)-N,N'-diacetic acid;
N,N'-bis(pyridoxal-5-phosphate)-1,2-isopropylene-N,N'-diacetic acid;
N,N'-bis(pyridoxal-5-phosphate)-1,2-(n-butylene)-N,N'-diacetic acid;
N,N'-bis(pyridoxal-5-phosphate)-1,4-(n-butylene)-N,N'-diacetic acid;
N,N'-bis(pyridoxal-5-phosphate)-1,3-(n-butylene)-N,N'-diacetic acid;
N,N'-bis(pyridoxal-5-phosphate)-1,2-(3-methylene)propyl-N,N'-diacetic acid;
N,N'-bis(pyridoxal-5-phosphate)-trans-1,2-cyclopentylenediamine-N,N'-diacetic acid;
N,N'-bis(pyridoxal-5-phosphate)-trans-1,2-cycloheptylenediamine-N,N'-diacetic acid;
N,N'-bis(pyridoxal-5-phosphate)-trans-1,2-cyclooctylenediamine-N,N'-diacetic acid;
N,N'bis(pyridoxal-5-phosphate)-1,2-phenylenediamine-N,N'-diacetic acid;
N,N'-bis(pyridoxal-5-phosphate)-cis-1,2-cyclohexylenediamine-N,N'diacetic acid;
N,N'-bis(pyridoxal-5-phosphate-(N-methylethanolamine)monoester)-ethylenediamine-N,N'-diacetic acid;
N,N'-bis(pyridoxal-5-phosphate(N-methylethanolamine)monoester)-1,3-(n-propylene)diamine- N,N'-diacetic acid;
N,N'-bis(pyridoxal-5-phosphate(N-methylethanolamine)monester)-1,2-(n-propylene)diamine-N,N'diacetic acid;
N,N'-bis(pyridoxal-5-phosphate(N-methylethanolamine)monoester)-1,2-isopropylenediamine-N,N'diacetic acid;
N,N'-bis(pyridoxal-5-phosphate(N-methylethanolamine)monoester)-1,2-(n-butylene)diamine-N,N'diacetic acid;
N,N'-bis(pyridoxal-5-phosphate(N-methylethyanolamine)monoester)-1,4-(n-butylene)diamine-N,N'-diacetic acid;
N,N'-bis(pyridoxal-5-phosphate(N-methylethanolamine)monoester)-1,3-(n-butylene)diamine-N,N'-diacetic acid;
N,N'-bis(pyridoxal-5-phosphate(N-methylethanolamine)monoester)-1,2,(3-methyl)propylenediamine-N,N'-diacetic acid;
N,N'bis(pyridoxal-5-phosphate(N-methylethanolamine)monoester)-trans-1,2-cyclohexylenediamine-N,N'-diacetic acid;
N,N'-bis(pyridoxal-5-phosphate(N-methylethanolamine)monoester)-trans-1,2-cyclopentylenediamine-N,N'-diacetic acid;
N,N'-bis(pyridoxal-5-phosphate(N-methylethanolamine)monoester)-trans-1,2-cycloheptylenediamine-N,N'-diacetic acid;
N,N'-bis(pyridoxal-5-phosphate(N-methylethanolamine)monoester)-trans-1,2-cyclooctylenediamine-N,N'-diacetic acid;
N,N'-bis(pyridoxal-5-phosphate(N-methylethanolamine)monoester)-1,2-phenylenediamine-N,N'-diacetic acid;
N,N'-bis(pyridoxal-5-phosphate(N-methylethanolamine)monester)-cis-1,2-cyclohexylenediamine-N,N'-diacetic acid;
N,N'-bis-(pyridoxal-5-phosphate)ethylenediamine-N-acetic acid;
N,N'-bis(pyridoxal-5-phosphate)-1,3-(n-propylene)diamine-N-acetic acid;
N,N'-bis(pyridoxal-5-phosphate)-1,2-(n-propylene)diamine-N-acetic acid;
N,N'-bis(pyridoxal-5-phosphate)-1,2-isopropylenediamine-N-acetic acid;
N,N'-bis(pyridoxal-5-phosphate)-1,2-(n-butylene)diamine-N-acetic acid;
N,N'-bis(pyridoxal-5-phosphate)-1,4-(n-butylene)diamine-N-acetic acid;
N,N'-bis(pyridoxal-5-phosphate)-1,3-(n-butylene)diamine-N-acetic acid;
N,N'-bis(pyridoxal-5-phosphate)-1,2-(3-methyl)propylenediamine-N-acetic acid;
N,N'-bis(pyridoxal-5-phosphate)-trans-1,2-cyclohexylenediamine-N-acetic acid;
N,N'bis(pyridoxal-5-phosphate)-trans-1,2-cyclopentylenediamine-N-acetic acid;
N,N'-bis(pyridoxal-5-phosphate)-trans-1,2-cycloheptylenediamine-N-acetic acid;
N,N'-bis(pyridoxal-5-phosphate)-trans-1,2-cyclooctylenediamine-N-acetic acid;
N,N'-bis(pyridoxal-5-phosphate)-1,2-phenylenediamine-N-acetic-acid;
N,N'-bis(pyridoxal-5-phosphate)-cis-1,2-cyclohexylenediamine-N-acetic acid;
N-pyridoxal-N'-(pyridoxal-5-phosphate)ethylenediamine-N,N'-diacetic acid (DPMP);
N,N'-bis(pyridoxal)-1,3-(n-propylene)-N,N'-diacetic acid;
N,N'-bis(pyridoxal)-1,2-(n-propylene)-N,N'-diacetic acid;
N,N'-bis(pyridoxal)-1,2-isopropylene-N,N'-diacetic acid;
N,N'-bis(pyridoxal)-1,2-(n-butylene)-N,N'-diacetic acid;
N,N'-bis(pyridoxal)-1,4-(n-butylene)-N,N'-diacetic acid
N,N'-bis(pyridoxal)-1,2-(3-methylene)propyl-N,N'-diacetic acid;
N,N'-bis-(pyridoxal)-trans-1,2-cyclohexylenediamine-N,N'-diacetic acid;
N,N'-bis(pyridoxal)-trans-1,2-cyclopentylenediamine-N,N'-diacetic acid;
N,N'-bis(pyridoxal)-trans-1,2-cycloheptylenediamine-N,N'-diacetic acid;
N,N'-bis(pyridoxal)-trans-1,2-cyclooctylenediamine-N,N'-diacetic acid;
N,N'-bis(pyridoxal)-1,2-phenylenediamine-N,N'-diacetic acid; and
N,N'-bis(pyridoxal)-cis-1,2-cyclohexylenediamine- N,N'-diacetic acid.

Particularly prferred chelating compounds of Formula I include:
N,N'-bis-(pyridoxal-5-phosphate)ethylene-diamine-N,N'-diacetic acid (referred to hereinafter as DPDP); and
N,N'-bis(pyridoxal-5-phosphate)-trans-1,2-cyclohexyldiamine-N,N'-diacetic acid (referred to hereinafter as DPCP); and
N,N'-bis-(pyridoxal)-ethylenediamine-N,N-diacetic acid.

Another group of suitable chelating compounds include compounds of Formula II

N(CH₂X)₃ (II)

(wherein X represents a -COOY, PO₃HY or -CONHOY group and Y represents a hydrogen atom, a metal ion equivalent and/or a basic biocompatible cation of an inorganic or organic base or basic amino acid)
and compounds of Formula III 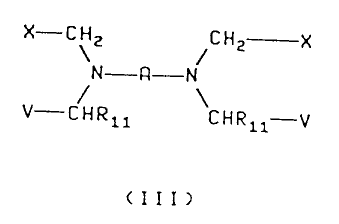
(wherein
X is as hereinbefore defined;
A represents a group

-CHR₁₂-CHR₁₃-,

-CH₂CH₂(ZCH₂-CH₂)ₘ,

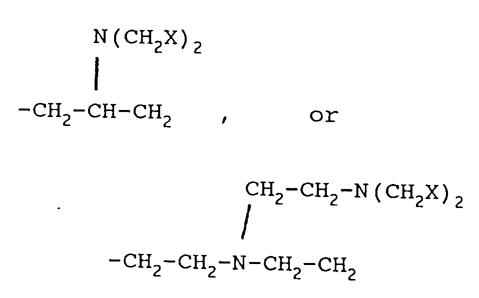

wherein X is as defined above;
each of the groups R₁₁ represents a hydrogen atom or a methyl group;
R₁₂ and R₁₃, which may be the same or different, each represents a hydrogen atom or a C₁₋₈ alkyl, phenyl or benzyl group, or
R₁₂ and R₁₃ together represent a C₁₋₈ alkylene group (e.g. trimethylene or tetramethylene etc);
m represents an integer from 1 to 3;
Z represents an oxygen or sulfur atom or a group

〉N-CH₂X or 〉N-CH₂-CH₂-OR₁₄

wherein X is as defined above;
R₁₄ represents a C₁₋₈ alkyl group;
Each of the groups V which may be the same or different represents a group X or a group

-CH₂OH or -CONH(CH₂)ₙX,

wherein X as defined above and
n represents an integer of from 1 to 12;
or if R₁₁, R₁₂ and R₁₃ represent hydrogen atoms, both the groups V together represent the group 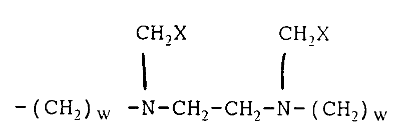
wherein X is as defined above, and W represents an integer of from 1 to 3).

By way of example suitable chelating compounds of Formula II include nitrilotriacetic acid and suitable chelating compounds of Formula III include trans-1,2-cyclohexylenediamine-N,N,N',N'-tetraacetic acid;
1,2-ethylenediamine-N,N,N'N'-tetramethanesphosphonic acid;
1,4,7,10-tetraazacyclododecane-N,N',N'',N'''-tetraacetic acid (DOTA);
N,N'-bis(1-hydroxybenzyl)ethylenediamine-N,N'-diacetic acid;
13,23-dioxo-15,18,21-tris(carboxymethyl)-12,15,18,21,24-pentaaza-pentatriacontanedioic acid;
3,9-bis(1-carboxyethyl)-6-carboxymethyl-3,6,9-triazundecanedioic acid;
ethylenedinitrilotetra(acetohydroxamic acid);
1,10-diaza-4,7-dithiadecane-1,1,10,10-tetraacetic acid;
1,2-diphenylethylenediaminetetraacetic acid;
N'-(2-hydroxyethyl)ethylenediamine-N,N,N'-triacetic acid(HEDTA);
1,4,8,11-tetraazacyclotetradecane-N,N',N''N'''-tetraacetic acid;
1,4,7,10-tetraazacyclododecane-N,N',N''-triacetic acid; diisopropyl iminodiacetic acid;
diethylenetrinitrilopenta(methylphosphonic acid); and 1-phenylethylenediaminetetracetic acid.
Especially preferred compounds of Formula II include diethylenetriaminepentaacetic acid (DTPA) and its analogs.

Compounds of Formula II and processes for their preparation are known and are described in US-A-4647447 and US-A-4639365.

A further group of suitable chelating compounds are those of Formula IV. 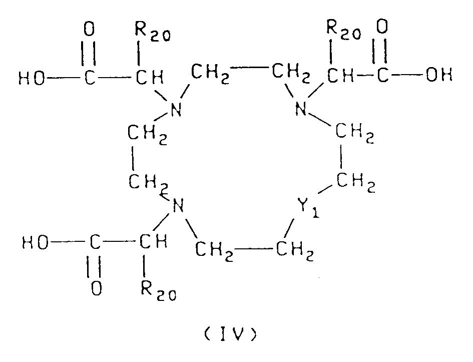
(wherein,
Y₁ represents an oxygen atom or a group

〉N-R₂₁ ,

R₂₀ represents a hydrogen atom or an alkyl, arylalkyl or aryl group; and
R₂₁ represents a hydrogen atom or an alkyl, hydroxyalkyl or carboxyalkyl group), and physiologically tolerable salts thereof.
In Formula IV, alkyl moieties of R₂₀ or R₂₁ preferably contain 1 to 5 carbon atoms, and especially preferably are methyl and aryl moieties are preferably optionally substituted phenyl, e.g., halo, alkyl, alkoxy, hydroxyl, carbamoyl or carboxyl substituted phenyl.
Preferred compounds of Formula IV include those wherein R₂₀ is hydrogen, and Y₁ is carboxymethylimino (DOTA);
wherein R₂₀ is hydrogen, and Y₁ is alkylimino, preferably methylimino (DO3A);
4,7,10-triscarboxymethyl-1-oxo-4,7,10-triazacyclododecane(DOXA);
1,4,7-triscarboxymethyl-1,4,7,10-tetraazacyclododecane;
1-methyl-4,7,10-triscarboxymethyl-1,4,7,10-tetraazacyclododecane; and
1-benzyl-4,7,10-triscarboxymethyl-1,4,7,10-tetraazacyclododecane.

Chelating compounds of Formula III and methods for their preparation are described in EP-A-232751(Squibb). DOTA and its preparation are described in US-A-4,647,447.

Especially preferred chelating compounds for use in accordance with the invention include DPDP, EDTP, DTPA, DCTA, DOXA, DO3A, and DOTA.

Other chelating agents proposed in the literature for the preparation of paramagnetic metal chelates for use as MRI contrast media may of course be used. In this regard the reader is referred particularly to recent publications of Nycomed, Squibb, Bracco, Schering and Guerbet such as WO89/00557, EP-A-292689, EP-A-230893, EP-A-255471, EP-A-277088 and EP-A-287465.

Using the Mn(II) chelates prepared according to the present invention contrast media may be formulated with conventional pharmaceutical or veterinary formulation aids, for example stabilizers, antioxidants, osmolality adjusting agents, buffers, pH adjusting agents, etc., and may be in a form suitable for parenteral or enteral administration directly into a body cavity having an external escape duct, for example the gastrointestinal tract, the bladder or the uterus. Thus the contrast media of the present invention may be as tablets, capsules, powders, solutions, suspensions, dispersions, syrups, suppositories etc.; however, solutions, suspensions and dispersions in physiologically acceptable carrier media, for example water for injections, will generally be preferred.

The contrast media may therefore be formulated for administration using physiologically acceptable carriers or excipients in a manner fully within the skill of the art. For example, the manganese chelate, optionally with the addition of pharmaceutically acceptable excipients, may be suspended or dissolved in an aqueous medium, with the resulting solution or suspension then being sterilized. As mentioned above, suitable additives include, for example, physiologically biocompatible buffers (as for example, tromethamine hydrochloride), slight additions of other chelating agents (as, for example, diethylenetriaminepentaacetic acid) or, optionally, calcium or sodium salts (for example, calcium chloride, calcium ascorbate, calcium gluconate or calcium lactate).

If the contrast media are to be formulated in suspension form, e.g. in water or physiological saline for oral administration, a small amount of an insoluble chelate salt may be mixed with one or more of the inactive ingredients conventionally present in oral solutions and/or surfactants and/or aromatics for flavouring.

The diagnostic media conveniently contain from 0.001 to 5.0 moles per liter and preferably from 0.1 to 0.5 moles per liter of the manganese chelate.

The chelates are administered to patients for imaging in amounts which are sufficient to yield the desired contrast. Generally, dosages of from 0.001 to 5.0 mmoles of contrast agent per kilogram of patient bodyweight are effective to achieve reduction of relaxivity rates. The preferred dosages for most MRI applications are from 0.05 to 0.5 mmoles of contrast agent per kilogram of patient bodyweight.

For MRI imaging of some portions of the body the most preferred mode for administering metal chelates as contrast agents is parenteral, e.g. intravenous administration. Parenterally administrable forms, e.g. intravenous solutions, should be sterile and free from physiologically unacceptable agents, and should have low osmolality to minimize irritation or other adverse effects upon administration and thus the contrast medium should preferably be isotonic or slightly hypertonic. Suitable vehicles include aqueous vehicles customarily used for administering parenteral solutions such as Sodium Chloride Injection, Ringer's Injection, Dextrose Injection, Dextrose and Sodium Chloride Injection, Lactated Ringer's Injection and other solutions such as are described in REMINGTON'S PHARMACEUTICAL SCIENCES, 15th ed., Easton: Mack Publishing Co, pp 1405-1412 and 1461-1487 (1975) and THE NATIONAL FORMULARY XIV, 14th ed. Washington: American Pharmaceutical Association (1975). The solutions can contain preservatives, antimicrobial agents, buffers and antioxidants conventionally used in parenteral solutions, excipients and other additives which are compatible with the chelates and which will not interfere with the manufacture, storage or use of products.

The contrast media may also, of course, be in concentrated or dried form for dilution prior to administration

Methods for applying MRI contrast media to improve MR images, MRI equipment and MRI operating procedures are described by Valk et al., BASIC PRINCIPLES OF NUCLEAR MAGNETIC RESONANCE IMAGING, New York: Elsevier, pp 109-114 (1985).

This invention is further illustrated by the following non-limiting Examples. Temperatures are given in degrees centigrade and concentrations as weight percentages unless otherwise specified.

### EXAMPLE 1

### MnDPDP Precipitate

H₈DPDP.2H₂O (1.00 g, 1.48 mmoles) and MnO (0.104 g, 1.46 mmoles) were slurried in 10 mL of water containing ascorbic acid (0.002 g, 0.01 mmoles) in a 20 mL erlenmeyer flask. The head space was purged with nitrogen, and the flask sealed with a rubber septum. Within 2 hours, the solution turned yellow, and no MnO particles were observed. The reaction was allowed to stir overnight. The yellow product was isolated by filtration and dried to a constant weight. A quantitative yield of product was obtained (MnDPDP, MW 691 g/mole).

The characterization follows:
Thermal Gravimetric Analysis (TGA): (30-270°C. 10°/min, N₂).
Calculation for trihydrate: 7.2%; found 8.2% (average of two runs).
Analysis calculated and (found) for
C₂₂H₃₀MnN₄0₁₄P₂.3H₂O: C, 35.44 (35.08); H, 4.83 (5.03); N, 7.52 (7.40); Mn, 7.37 (7.36)
Negative ion liquid secondary ion mass spectrometry (n-LSIMS) calculated for [M-1]⁻peak and (found): m/z 690 (690).

### EXAMPLE 2

### MnEDTP Precipitate

H₈EDTP (1.165 g, 2.50 mmoles) and MnO (0.176 g, 2.50 mmoles) were slurried in 10 mL of distilled water. The suspension was stirred and turned white after four hours. Solid H₆MnEDTP (MW = 489.05 gm/mole) was isolated by filtration and washed with water and methanol before drying at 50°C. A quantitative yield of product was obtained.

The characterization follows:
Negative ion liquid secondary ion mass spectrometry (n-LSIMS) calculated and (found) for [M-1]⁻ peak: m/z 488 (488).
Osmolality at pH 6.5 (normalized to 1 M): 3.26 Osmoles/kg.

### EXAMPLE 3

### MnDTPA Precipitate

H₅DTPA (0.983 g, 2.50 mmoles) and MnO (0.177 gm, 2.50 mmoles) were slurried in 10 mL of distilled water. The suspension was stirred and turned green in colour after four hours. After stirring for an additional eight hours, the suspension turned white. Solid H₃MnDTPA (MW = 446.28 gm/mole) was isolated by filtration and washed with water and methanol before drying at 50^{o}C. A 50% yield of product was obtained.

The characterization follows: Negative ion liquid secondary ion mass spectrometry (n-LSIMS) calculated and (found) for [M-1]⁻ peak: M/z 445 (445).
Osmolality at pH 6.5 (normalized to 1 M): 2.98 Osmoles/kg.

### EXAMPLE 4

### MnDCTA Precipitate

H₄DCTA (0.911 g, 2.50 mmoles) and MnO (0.176 g, 2.48 mmoles) were slurried in 10 mL of distilled water. The suspension was stirred and turned red in color after four hours. After stirring for an additional eight hours, the suspension had a slight pink color which turned to a completely white suspension upon heating at 45^{o}C for ten minutes. Solid H₂MnDCTA (MW = 399.26 gm/mole) was isolated by filtration and washed with water and methanol before drying at 50^{o}C. A 25% yield of product was obtained.

The characterization follows:
Negative ion liquid secondary ion mass spectrometry (n-LSIMS) calculated and (found) for [M-1]⁻ peak: m/z 398 (398).
Osmolality at pH 6.5 (normalized to 1 M): 2.56 Osmoles/kg.

### EXAMPLE 5

### Osmolarity Values

The osmolarity values for 1 M solutions prepared with MnO compared to those prepared with MnCl₂ are summarized as follows:

| Compound | Prepared From | | | |
|---|---|---|---|---|
| | MnCl₂ | | MnO | |
| | Theory^{a} | Found | Theory | Found |
| MnDPDPHₓNa₃₋ₓ | 6-8 | 7.6 | 2-4 | 3.3 |
| MnDCTAHₓNa₂₋ₓ | 6-7 | - | 2-3 | 2.6 |
| MnDTPAHₓNa₃₋ₓ | 7-8 | - | 3-7 | 3.0 |
| MnEDTPHₓNa₆₋ₓ | 7-11 | - | 3-7 | 3.3 |

| | | | | |
|---|---|---|---|---|
| ^{a} Actual values depend upon solution pH. | | | | |

## Claims

1. A process for the preparation of a Mn(II) chelate which process comprises reacting MnO in an aqueous medium at a pH of no more than 5 and a temperature of from 20 to 50°C with a chelating compound other than EDTA which chelating compound is substantially water-insoluble and is substantially protonated whereby at least 75% of the labile protons in said chelating compound in its fully protonated form are present, and optionally subsequently dissolving precipitated Mn(II) chelate in an aqueous base.

2. A process as claimed in claim 1 wherein the MnO is reacted with at least one molar equivalent of the chelating compound.

3. A process as claimed in either of claims 1 and 2 comprising separating precipitated Mn(II) chelate and dissolving the separated precipitate in a physiologically tolerable aqueous base solution.

4. A process as claimed in any one of claims 1 to 3 wherein said chelating compound is a chelating compound of Formula I 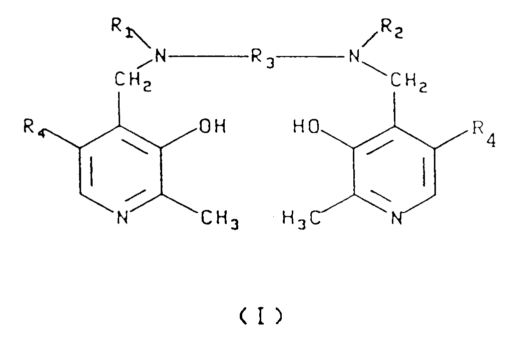 (wherein
R₁ represents a hydrogen atom or a group 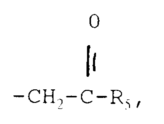 and
R₂ represents a hydrogen atom or a group 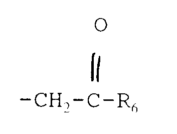 with the proviso that one of the groups R₁ and R₂ is other than hydrogen;
R₃ represents a C₁₋₈ alkylene, C₅₋₈ 1,2-cycloalkylene, or C₆₋₁₀ 1,2-arylene group;
each of the groups R₄ which may be the same or different represents a hydrogen atom, a C₁₋₆ alkyl group or a group -CH₂OH, or a group 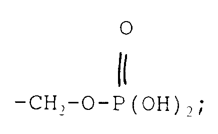 with the proviso that where one R₄ represents hydrogen or alkyl the other represents a group -CH₂OP(O)(OH)₂; R₅ and R₆ which may be the same or different each represents a hydroxy, C₁₋₁₈, optionally hydroxy-substituted, alkoxy, amino or C₁₋₈ alkylamino group),
and pharmaceutically acceptable salts, and where group R₄ represents a group CH₂-O-PO(OH)₂, the phosphate group mono and diesters thereof with mono and polyhydric C₁₋₁₈ alkyl- or alkylamino alcohols.

5. A process as claimed in any one of claims 1 to 4 wherein said chelating compound is a chelating compound of Formula II
N(CH₂X)₃ (II)
(wherein X represents a -COOY, PO₃HY or -CONHOY group and Y represents a hydrogen atom, a metal ion equivalent and/or a basic biocompatible cation of an inorganic or organic base or basic amino acid), or a physiologically tolerable salt thereof.

6. A process as claimed in any one of claims 1 to 3 wherein said chelating compound is a chelating compound of Formula III 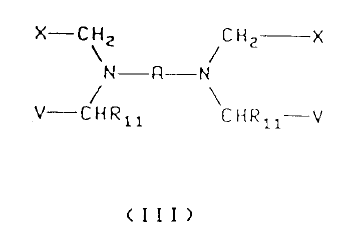 (wherein
A represents a group
-CHR₁₂-CHR₁₃-,
-CH₂CH₂(ZCH₂-CH₂)ₘ, 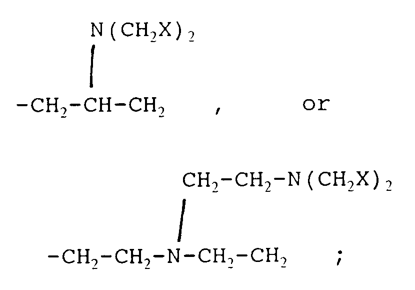
each of the groups R₁₁ represents a hydrogen atom or a methyl group;
R₁₂ and R₁₃, which may be the same or different, each represents a hydrogen atom or a C₁₋₈ alkyl, phenyl or benzyl group, or
R₁₂ and R₁₃ together represent a C₁₋₈ alkylene group
m represents an integer, from 1 to 3;
Z represents an oxygen or sulfur atom or a group
〉N-CH₂X or 〉N-CH₂-CH₂-OR₁₄
R₁₄ represents a C₁₋₈ alkyl group;
Each of the groups V which may be the same or different represents a group X or a group
-CH₂OH or -CONH(CH₂)ₙX
and n represents an integer of from 1 to 12,
or if R₁₁, R₁₂ and R₁₃ represent hydrogen atoms, both the groups V together represent the group 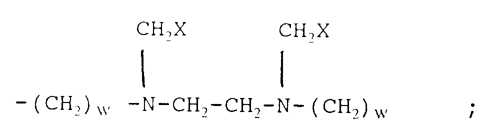 w represents an integer of from 1 to 3; and X is as defined in claim 5),
or a physiologically tolerable salt thereof.

7. A process as claimed in any one of claims 1 to 3 wherein said chelating compound is a chelating compound of Formula IV 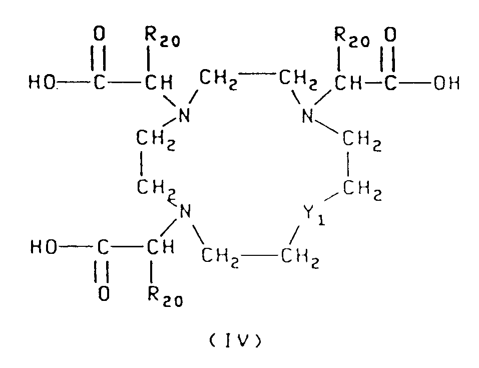 (wherein
Y₁ represents an oxygen atom or a group
〉N-R₂₁ ,
R₂₀ represents a hydrogen atom or an alkyl, arylalkyl or aryl group; and
R₂₁ represents a hydrogen atom or an alkyl, hydroxyalkyl or carboxyalkyl group), or a physiologically tolerable salt thereof.

8. A process as claimed in any one of claims 1 to 3 wherein said chelating compound is a chelating compound selected from DPDP, DTPA, DCTA, EDTP, DOXA, DO3A, and DOTA.

## Patentansprüche

1. Verfahren zur Herstellung eines Mn(II)-Chelates, umfassend das Umsetzen von MnO in einem wäßrigen Medium bei einem pH-Wert von nicht mehr als 5 und einer Temperatur von 20 bis 50°C mit einer anderen Chelatisierungsverbindung als EDTA, wobei die Chelatisierungsverbindung im wesentlichen wasserunlöslich und im wesentlichen protoniert ist, wobei wenigstens 75% der labilen Protonen in der Chelatisierungsverbindung in ihrer vollständig protonierten Form vorhanden sind, und wahlweise das nachfolgende Auflösen des präzipitierten Mn(II)-Chelates in einer wäßrigen Base.

2. Verfahren gemäß Anspruch 1, worin das MnO mit wenigstens einem Moläquivalent der Chelatisierungsverbindung umgesetzt wird.

3. Verfahren gemäß Anspruch 1 oder 2, umfassend das Abtrennen des präzipitierten Mn(II)-Chelats und Auflösen des abgetrennten Präzipitats in einer physiologisch verträglichen wäßrigen Base-Lösung.

4. Verfahren gemäß einem der Ansprüche 1 bis 3, worin die Chelatisierungsverbindung eine Chelatisierungsverbindung der Formel I ist: 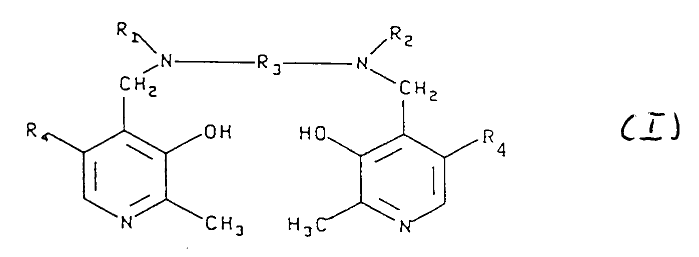 worin
R₁ ein Wasserstoffatom oder eine Gruppe 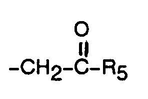 darstellt und
R₂ ein Wasserstoffatom oder eine Gruppe 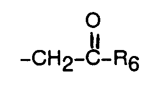 darstellt, mit der Maßgabe, daß eine der Gruppen R₁ und R₂ nicht Wasserstoff ist,
R₃ eine C₁₋₈-Alkylen-, eine C₅₋₈-1,2-Cycloalkylen- oder eine C₆₋₁₀-1,2-Arylengruppe bedeutet,
worin jede der Gruppen R₄ gleich oder verschieden sein können und ein Wasserstoffatom, eine C₁₋₆-Alkylgruppe oder eine -CH₂OH-Gruppe oder eine Gruppe 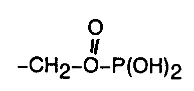 darstellt, mit der Maßgabe, daß wenn ein Rest R₄ Wasserstoff oder Alkyl bedeutet, dann der andere Rest eine Gruppe -CH₂OP(O) (OH)₂ darstellt; worin
R₅ und R₆ gleich oder verschieden sein können und jeweils eine Hydroxy-, eine C₁₋₁₈, wahlweise hydroxy-substituiert, Alkoxy-, Amino- oder eine C₁₋₈-Alkylaminogruppe bedeuten,
und pharmazeutisch verträgliche Salze und, wenn die Gruppe R₄ eine CH₂-O-PO(OH)₂-Gruppe bedeutet, die Phosphonat-Mono- und -Diester hiervon mit mono- und polyhydroxylischen C₁₋₁₈-Alkyl- oder Alkylamino-Alkoholen.

5. Verfahren gemäß einem der Ansprüche 1 bis 4, worin die Chelatisierungsverbindung eine Chelatisierungsverbindung der Formel II
N(CH₂X)₃ (II)
ist, worin X eine -COOY-, PO₃HY- oder -CONHOY-Gruppe bedeutet und Y ein Wasserstoffatom, ein Metallionäquivalent und/oder ein basisches biokompatibles Kation einer anorganischen oder organischen Base oder basischen Aminosäure bedeutet, oder ein physiologisch verträgliches Salz hiervon.

6. Verfahren gemäß einem der Ansprüche 1 bis 3, worin die Chelatisierungsverbindung eine Chelatisierungsverbindung der Formel III ist 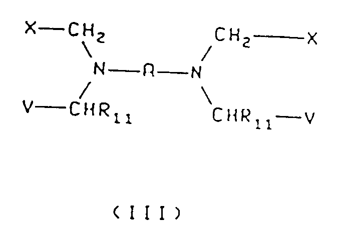 worin
A eine Gruppe
-CHR₁₂-CHR₁₃-,
-CH₂CH₂(ZCH₂-CH₂)ₘ, 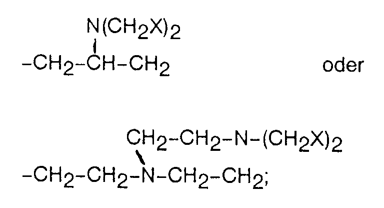
bedeutet, worin jede der Gruppen R₁₁ ein Wasserstoffatom oder eine Methylgruppe bedeuten;
R₁₂ und R₁₃ gleich oder verschieden sein können und jeweils ein Wasserstoffatom oder eine C₁₋₈-Alkylgruppe, Phenyl oder Benzyl bedeuten,
R₁₂ und R₁₃ zusammen eine C₁₋₈-Alkylengruppe bedeuten,
m eine Zahl von 1 bis 3 darstellt,
Z ein Sauerstoff- oder Schwefelatom oder eine Gruppe
N-CH₂X oder N-CH₂-CH₂-OR₁₄
darstellt,
R₁₄ eine C₁₋₈-Alkylgruppe bedeutet;
jede der Gruppen V gleich oder verschieden sein können und eine Gruppe X oder eine Gruppe
-CH₂OH oder -CONH(CH₂)ₙX
darstellt,
und n eine Zahl von 1 bis 12 bedeuten,
oder, wenn R₁₁, R₁₂ und R₁₃ Wasserstoffatome darstellen, beide Gruppen V zusammen die Gruppe 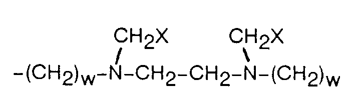 bedeuten,
w eine Zahl von 1 bis 3 ist und X wie in Anspruch 5 definiert ist,
oder ein physiologisch verträgliches Salz hiervon.

7. Verfahren gemäß einem der Ansprüche 1 bis 3, worin die Chelatisierungsverbindung eine Chelatisierungsverbindung der Formel IV 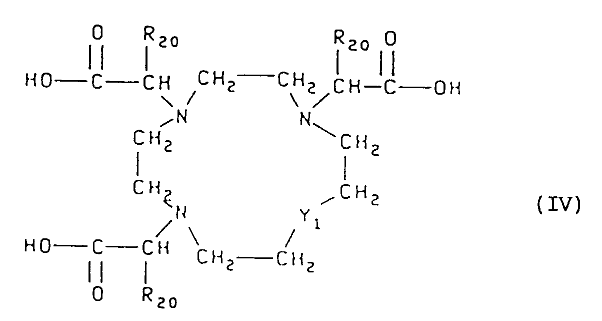 ist, worin
Y₁ ein Sauerstoffatom oder eine Gruppe
N-R₂₁
darstellt,
R₂₀ ein Wasserstoffatom oder eine Alkyl-, Arylalkyl- oder Arylgruppe bedeutet und
R₂₁ ein Wasserstoffatom oder eine Alkyl-, Hydroxyalkyl oder Carboxyalkylgruppe bedeutet,
oder ein physiologisch verträgliches Salz hiervon.

8. Verfahren gemäß einem der Ansprüche 1 bis 3, worin die Chelatisierungsverbindung eine Chelatisierungsverbindung ausgewählt aus DPDP, DTPA, DCTA, EDTP, DOXA, DO3A und DOTA ist.

## Revendications

1. Procédé de préparation d'un composé chélate du Mn(II) lequel procédé comprend la réaction de MnO dans un milieu aqueux à un pH d'au plus 5 et une température comprise entre 20 et 50° C avec un composé chélatant autre que l'EDTA lequel composé chélatant est pratiquement insoluble dans l'eau et est pratiquement protoné ce par quoi au moins 75 % des protons labiles sont présents dans ledit composé chélatant dans sa forme complètement protonée, et éventuellement par la suite la dissolution du composé chélate du Mn(II) précipité dans une base aqueuse.

2. Procédé selon la revendication 1 dans lequel on fait réagir MnO avec au moins un équivalent molaire du composé chélatant.

3. Procédé selon l'une quelconque des revendications 1 et 2 comprenant la séparation du composé chélate du Mn(II) précipité et la dissolution du précipité séparé dans une solution de base aqueuse physiologiquement tolérable.

4. Procédé selon l'une quelconque des revendications 1 à 3 dans lequel ledit composé chélatant est un composé chélatant de formule I 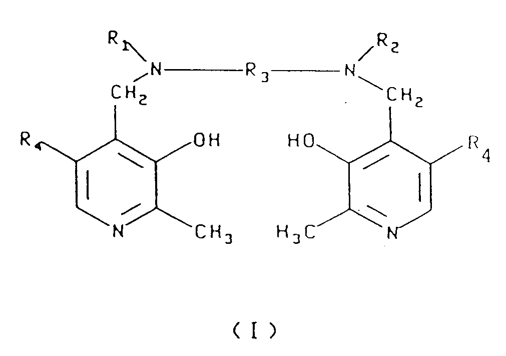 dans laquelle R₁ représente un atome d'hydrogène ou un groupe 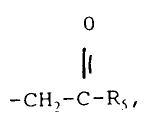 et
R₂ représente un atome d'hydrogène ou un groupe 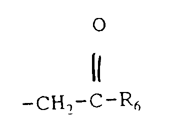 sous réserve qu'un des groupes R₁ et R₂ soit autre qu'un atome d'hydrogène ;
R₃ représente un groupe alkylène en C₁₋₈, 1,2-cycloalkylène en C₅₋₈, ou un groupe 1,2-arylène en C₆₋₁₀
chacun des groupes R₄ qui peuvent être identiques ou différents représentent un atome d'hydrogène, un groupe alkyle en C₁₋₆ ou un groupe -CH₂OH, ou un groupe 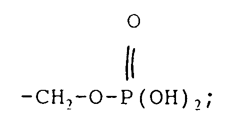 sous réserve que lorsqu'un des groupes R₄ représente un atome d'hydrogène ou un groupe alkyle l'autre représente un groupe -CH₂OP(O)(OH)₂ ;
R₅ et R₆ qui peuvent être identiques ou différents représentent chacun un groupe hydroxyle, un groupe alcoxy en C₁₋₁₈, éventuellement substitué par un groupe hydroxyle, un groupe amino ou un groupe (alkyle en C₁₋₈) amino, et sels pharmaceutiquement acceptables, et lorsque R₄ représente un groupe -CH₂-O-PO(OH)₂, les composés dans lesquels le groupe phosphate est sous forme de mono- ou diester avec des (alkyle en C₁₋₁₈)- ou (alkyle en C₁₋₁₈)amino- mono et polyalcools.

5. Procédé selon l'une quelconque des revendications 1 à 4 dans lequel ledit composé chélatant est un composé chélatant de formule II
N(CH₂X)₃ (II)
(dans laquelle X représente un groupe -COOY, PO₃HY ou - CONHOY et Y représente un atome d'hydrogène, un équivalent d'ion métallique et/ou un cation biocompatible de base d'une base inorganique ou organique ou d'un amino acide de base), ou un sel physiologiquement tolérable de ce dernier.

6. Procédé selon l'une quelconque des revendications 1 à 3 dans lequel ledit composé chélatant est un composé chélatant de formule III 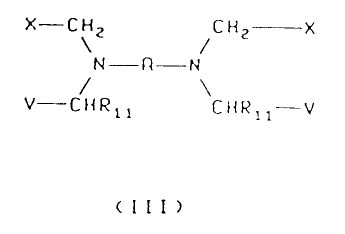 (dans laquelle A représente un groupe
-CHR₁₂-CHR₁₃-,
-CH₂CH₂(ZCH₂-CH₂)ₘ, 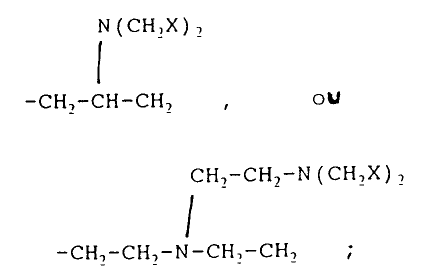
chacun des groupes R₁₁ représente un atome d'hydrogène ou un groupe méthyle ;
R₁₂ et R₁₃, qui peuvent être identiques ou différents, représentent chacun un atome d'hydrogène ou un groupe alkyle en C₁₋₈, phényle ou benzyle ou
R₁₂ et R₁₃ représentent ensemble un groupe alkylène en C₁₋₈
m représente un entier, compris entre 1 et 3
Z représente un atome d'oxygène ou de soufre ou un groupe
〉N-CH₂X ou 〉N-CH₂-CH₂-OR₁₄
R₁₄ représente un groupe alkyle en C₁₋₈ ;
chacun des groupes V qui peuvent être identiques ou différents représente un groupe X ou un groupe
-CH₂OH or -CONH(CH₂)ₙX
et n représente un entier compris entre 1 et 12,
ou si R₁₁, R₁₂ et R₁₃ représentent des atomes d'hydrogène, les deux groupes V représentent ensemble le groupe 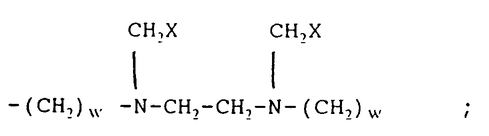 w représente un entier compris entre 1 et 3; et X est tel que défini dans la revendication 5),
ou un sel physiologiquement tolérable de ce dernier.

7. Procédé selon l'une quelconque des revendications 1 à 3 dans lequel ledit composé chélatant est un composé chélatant de formule IV 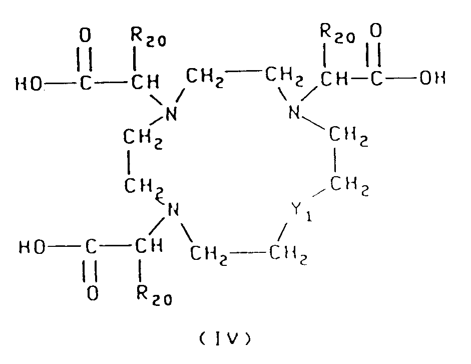 (dans laquelle Y₁ représente un atome d'oxygène ou un groupe
〉N-R₂₁ ,
R₂₀ représente un atome d'hydrogène ou un groupe alkyle, arylalkyle ou aryle ; et
R₂₁ représente un atome d'hydrogène ou un groupe alkyle, hydroxyalkyle ou carboxyalkyle), ou un sel physiologiquement tolérable de ce dernier.

8. Procédé selon l'une quelconque des revendications 1 à 3 dans lequel ledit composé chélatant est un composé chélatant choisi parmi les DPDP, DTPA, DCTA, EDTP, DOXA, DO3A, et DOTA.
